# EUROPEAN PATENT APPLICATION

(11) **EP 4 029 874 A2**
(43) Date of publication of application: **20.07.2022**
(21) Application number: 20803078.3
(22) Date of filing: 09.09.2020
(51) Int. Cl.: C07K 7/06

(54) **ANTI-INFLAMMATORY PEPTIDES, PHARMACEUTICAL COMPOSITION CONTAINING SUCH PEPTIDES, AND USES THEREOF**

(30) Priority: 09.09.2019 BR 102019018666
(71) Applicant: INSTITUTO BUTANTAN, 05503900 São Paulo - SP (BR)
(72) Inventor: TAVASSI, Ana Marisa Chudzinski, 05578-070 São Paulo - SP (BR); FLORES, Miryam Paola Alvarez, 05595-040 São Paulo - SP (BR); CURY, Yara, 06539-045 Santana de Parnaíba - SP (BR); BUFALO, Michelle Cristiane, 18520-000 Cerquilho - SP (BR); KERKIS, Irina, 05586-060 São Paulo - SP (BR); CERQUEIRA, Fernanda Menezes, 01224-030 São Paulo - SP (BR); FRARE, Eduardo Osorio, 05366-150 São Paulo - SP (BR)
(74) Representative: Nederlandsch Octrooibureau
(86) International application number: PCT/BR2020/050360
(87) International publication number: WO 2021/046626

(57) **Abstract**

The present invention relates to various sequences of new peptides and derivatives with anti-inflammatory effects in various cellular models (chondrocytes osteoclasts, macrophages derived from THP-1, and neurons) in the context of stimulus-induced inflammation widely known in the literature for each model. The compounds were shown to be able to reverse the expression of molecules associated with the cell's inflammatory mechanism, in addition to inducing regenerative proteins and reducing pain markers. Accordingly, the claimed compounds of the present invention can be applied to degenerative diseases of an inflammatory nature including rheumatoid arthritis and osteoarthritis.

## Description

### FIELD OF THE INVENTION

The present invention relates to various sequences of new peptides and derivatives with anti-inflammatory effects in various cellular models (macrophages derived from THP-1, neurons, osteoclasts, and chondrocytes), in the context of stimulus-induced inflammation widely known in the literature for each model. The compounds were shown to be able to reverse the expression of molecules associated with the cell's inflammatory mechanism, in addition to inducing regenerative proteins and reducing pain markers. Accordingly, the compound claimed in the present invention can be applied to degenerative diseases of an inflammatory nature including rheumatoid arthritis and osteoarthritis.

### BACKGROUND OF THE INVENTION

Osteoarthritis (OA) is a degenerative joint disease characterized by the degradation of the extracellular matrix (ECM) and the chondrocytes present in the cartilage (KAPOOR *et al.,* 2011). In patients who developed the disease, there is an increase in inflammatory mediators in synovial fluid, such as interleukins and nitric oxide (NO), which induce chondrocyte apoptosis (ZHOU *et al.,* 2008 & KAPOOR *et al.,* 2011 & AKKIRAJU; NOHE, 2015). Chondrocytes have receptors for ECM components (GOLDRING & OTERO, 2011), and this interaction between ECM and chondrocytes has a regulatory function that ensures cell survival, and when deregulated is capable of causing pain in patients since it leads to the loss of ECM and chondrocytes, in addition to the release of NO (PERROT, 2015) . The existing treatments do not reverse the disease status (AKKIRAJU; NOHE, 2015). Therefore, studies on new molecular targets are needed to find a new treatment for OA.

*Lonomia obliqua* is a Brazilian caterpillar known to cause hemorrhagic syndrome in patients who have had contact with its bristles (ZANNIN *et al*., 2003).

Some studies have identified the proteins responsible for the changes in blood clotting presented in patients. Interestingly, in addition to modulating blood coagulation, some of these molecules modulate the mechanisms of inflammation and cell survival when studied *in vitro* in cell models, having the ability to reduce cell death from stress induced by serum deprivation (ALVAREZ-FLORES *et al.,* 2006, 2011; FRITZEN *et al*., 2005; REIS *et al.,* 2001a, 2001b, REIS *et al*., 2006). These molecules have been extensively studied (structurally and functionally). These studies led to the design of smaller peptide entities that have anti-apoptotic activity, without pro-coagulant effects. The peptides corresponding to several conserved motifs, with or without changes in the sequences, corresponding to the cytoprotective molecules of the secretions of the *Lonomia obliqua* caterpillar.

Macrophages are cells of the immune system that have functions of phagocytosis and presentation of antigens. Macrophages produce inflammatory mediators and destructive responses during the osteoarthritis process (NI *et al.,* 2019). Considering that the injury process involves the inflammatory response, several anti-inflammatory screening models have been established, such as macrophages derived from the THP-1 strain.

Osteoclasts are multinucleated cells formed from cells of the monocyte/macrophage lineage, and their differentiation and function are regulated by various cytokines, hormones, and growth factors. In particular, the macrophage colony-stimulating factor (M-CSF), and the receptor activator for nuclear factor k-B ligand (RANKL) are essential in the regulation of the sequential osteoclast genesis process, including the proliferation of osteoclast precursors, adhesion, cell-cell migration, and fusion to form multinucleated cells, as well as in the migration, survival and bone resorption function of mature osteoclasts (LEE *et al*., 2018).

The Substance P (SP) is a peptide mainly secreted by neurons and is involved in many biological processes, including nociception (pain transmission process) and neurogenic inflammation (MASHAGHI *et al*., 2016).

β-endorphins are neuropeptides involved in pain control, having effects similar to morphine, and capable of suppressing inflammation and reducing the production of pro-inflammatory mediators in arthritis-induced inflammatory pain (Sprouse-Blum *et al.,* 2010; HE *et al*., 2018).

Thus, studies carried out on this invention have made it possible to determine several synthetic peptides with activity both in cell remodeling and in reducing inflammation and pain markers that make them candidates for new treatments, which are more specific and efficient for degenerative diseases of an inflammatory nature.

### BRIEF DESCRIPTION OF THE DRAWINGS

The structure and operation of the present invention, together with additional advantages thereof, can be better understood by reference to the attached drawings and the following description:

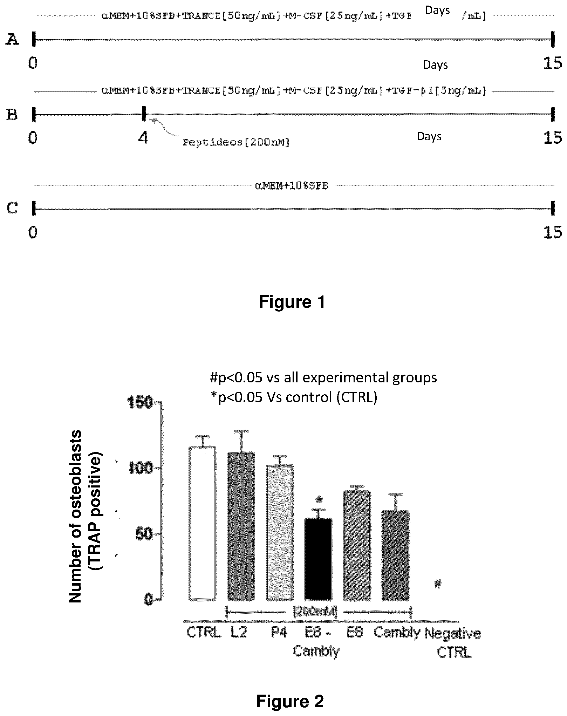

Figure 1 shows an experimental design of the osteoclast differentiation model and the screening of anti-inflammatory peptides. A. Positive differentiation controls. B. Influence of synthetic peptides (200 nM) on osteoclast differentiation. C. Negative differentiation control. αMEM - Minimum Essential Medium Eagle Alpha Modification; FBS - fetal bovine serum; TRANCE - Tumor Necrosis Factor (TNF)-related Activation-induced Cytokine; M-CSF - Macrophage Colony-Stimulating Factor; TGF-β1 - Transforming growth factor beta-1.
Figure 2 shows the number of TRAP-positive cells observed after treatments with synthetic peptides in comparison with positive and negative controls for osteoclast differentiation;

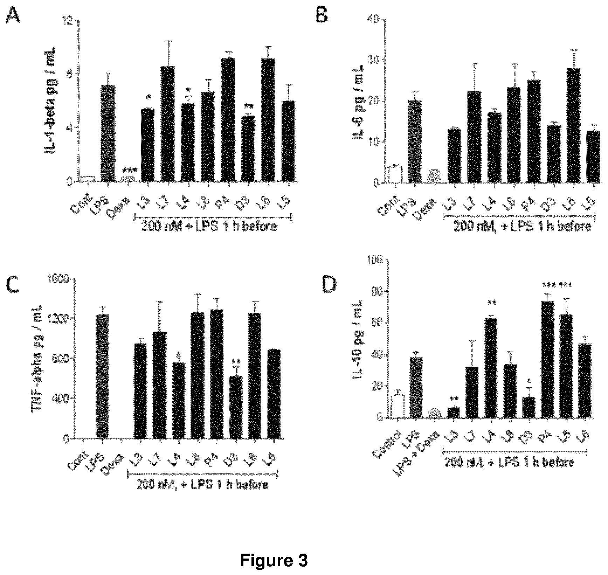

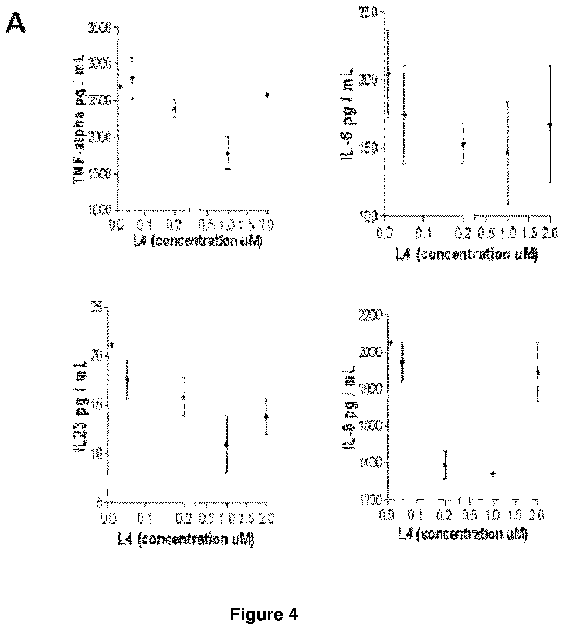

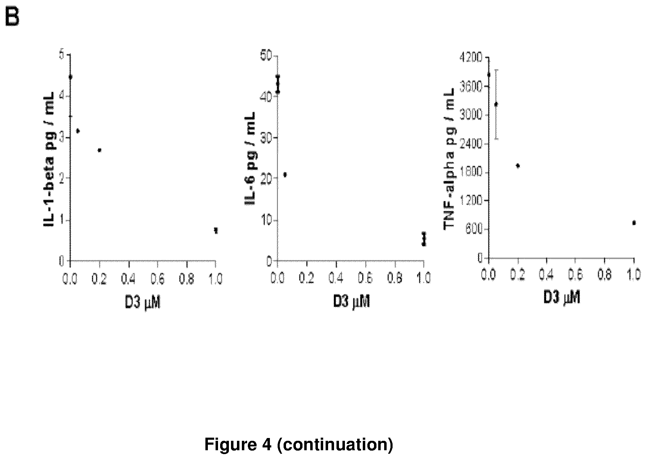

Figure 3 shows the anti-inflammatory effects of the synthetic peptide on the production of inflammatory cytokines (IL-1β, IL-6, and TNF-alpha) and the IL-10 anti-inflammatory cytokine. THP-1 cells differentiated into macrophages were induced with LPS (10 ng/mL) and, one hour later, treated with synthetic peptides at a concentration of 200 nM. Dexamethasone (10 µM), an inflammatory inhibitor, was used as a control. After 24 hours, the culture medium was collected to measure the IL-1β (A), IL-6 (B), TNF-α (C), and IL-10 cytokines using a Multiplex kit. * p < 0.05 vs. LPS; ** p < 0.01 vs. LPS; *** p < 0.005 vs. LPS;
Figure 4 shows the Dose-Response Effect of L4 and D3 peptide on the production of inflammatory cytokines. THP-1 cells differentiated into macrophages were induced with LPS (10 ng/mL) and, one hour later, treated with L4 or D3 peptide (0,05 µM, 0,2 µM, 1 µM, and 2 µM). After 24 hours, the culture medium was collected and TNF-alpha, IL-6, IL-23, and IL-8 were measured by Multiplex. (A) L4 peptide. (B) D3 peptide;
Figure 5 shows: (A) production of Substance P in neurons incubated with D3 peptide (200 nM and 500 nM) 3 hours before incubation with IL-1β (10 ng/mL); (B) Production of Substance P in neurons incubated with peptide D3 (200 nM and 500 nM) 3 hours after incubation with IL-1β (10 ng/mL); (C) production of β-endorphin in neurons incubated with peptides 3 hours after incubation with IL-1β (10 ng/mL). ANOVA and Dunnet ** p < 0.01, *** p < 0.001 compared to IL-1β;

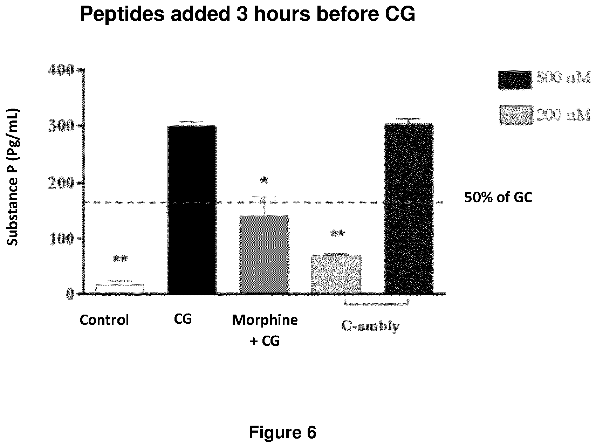

Figure 6 shows the production of substance P in neurons incubated with the Cambly peptide (200 nM and 500 nM) 3 hours before incubation with glycated collagen (GC). ANOVA and Dunnet * p < 0.05; ** p < 0.01 compared to glycated collagen (GC);

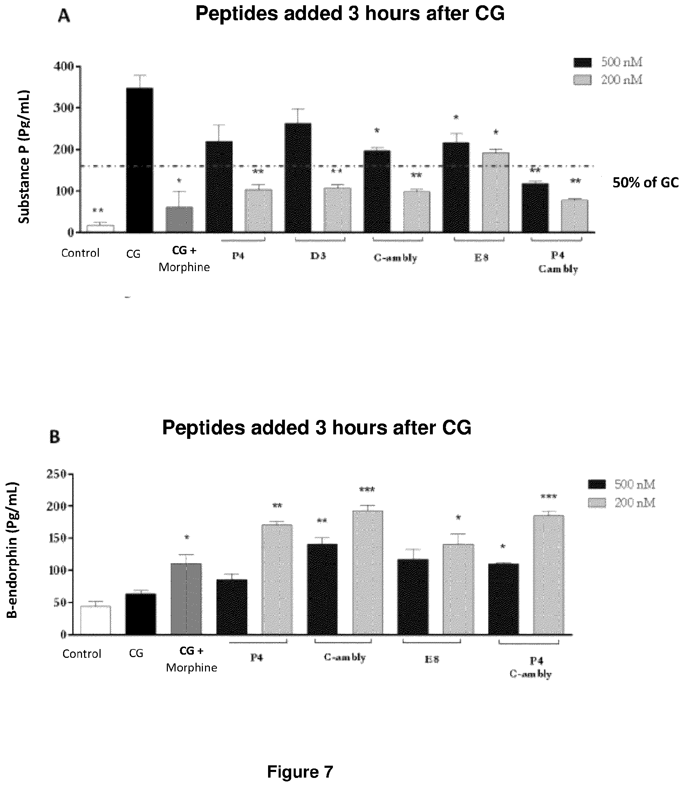

Figure 7 shows: (A) production of Substance P; and (B) production of β-endorphin in neurons incubated with peptides 3 hours after incubation with glycated collagen (GC). ANOVA and Dunnet * p < 0.05 ** p < 0.01, *** p < 0.001 compared with GC;

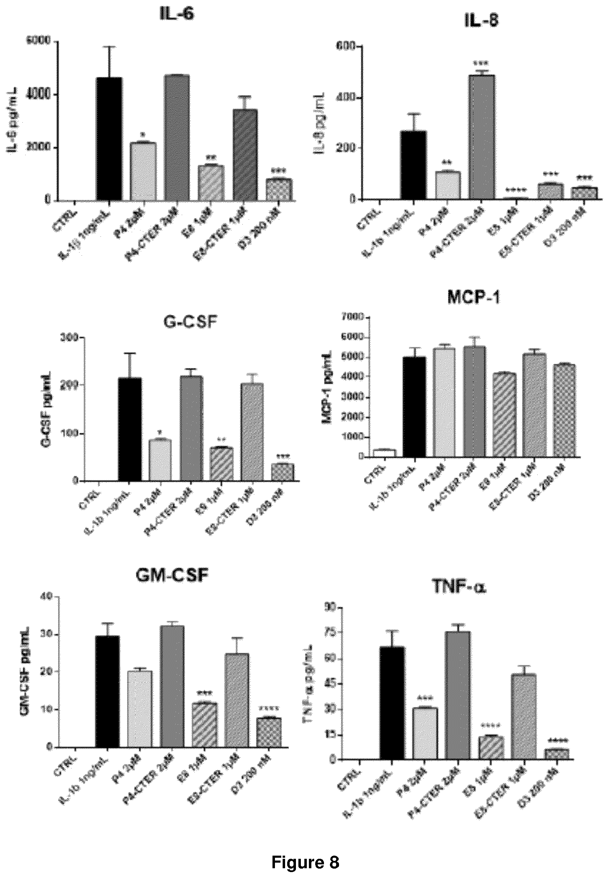

Figure 8 shows the effect of peptides on the release of 6 analytes in the pro-inflammatory model of chondrocytes treated with IL-1β (1 ng/mL). Values were obtained from each analyte in pg/mL from treatment with the peptides in the presence of IL-1β (1 ng/ mL). The data are presented as the mean ± MSE (* p < 0.05 when compared to the control with IL-1β, test t, n = 3).

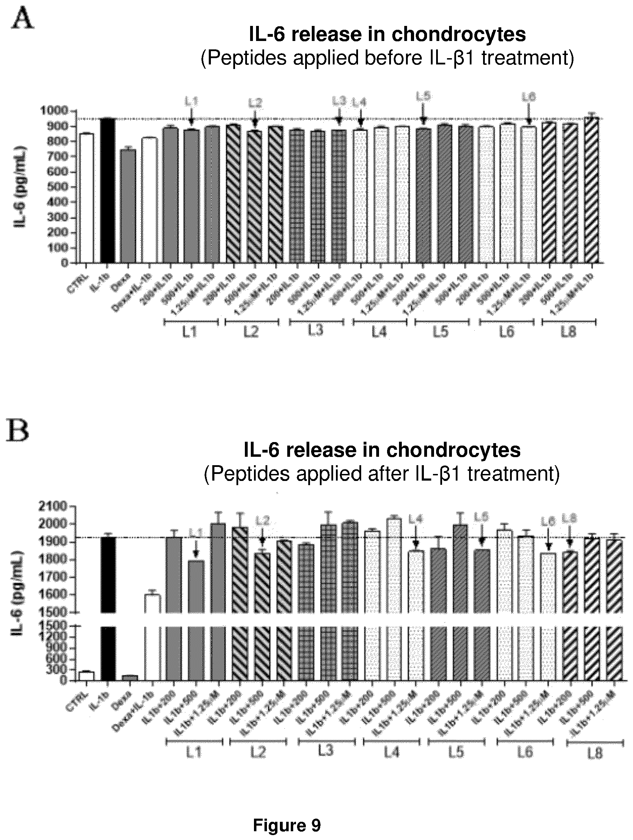

Figure 9 shows the effect of synthetic peptides on IL-6 release in the pro-inflammatory model of chondrocytes treated with IL-1β (A) chondrocytes were pretreated with peptide (200 nM, 500 nM, and 1.25 µM) for 1 hour before the addition of IL-1β (1 ng/mL) and an additional incubation of 24 hours before the collection of supernatants. (B) Chondrocytes were pretreated for 1 hour with IL-1β (1 ng/mL) before the addition of peptides (200 nM, 500 nM, and 1.25 µM) and an additional incubation of 24 hours before collecting the supernatants.

### DETAILED DESCRIPTION OF THE INVENTION

To solve the problems mentioned above, the present invention will provide significant advantages over the use of various sequences of new peptides and derivatives with anti-inflammatory effects in various cellular models (chondrocytes, osteoclasts, macrophages derived from THP-1, and neurons), in the context of stimulus-induced inflammation widely known in the literature for each model. Thus, enabling an increase in its performance and presenting a more favorable cost/benefit ratio.

### Material and methods

### Peptides

The present peptides can be chemically synthesized by standard methods of chemical synthesis widely known, including the solid phase methodology that uses polymeric supports, such as a p-methyl-benzhydryl amine resin (MBHA) for synthesis by the t-Boc or F-moc strategy (MACHADO *et al*., 2004) . The peptides can also be biologically synthesized by recombinant DNA technology using, for example, an expression vector containing a nucleic acid sequence encoding one of the peptides described in the present invention. After synthesis, the peptides can be purified by known methods, including high-performance liquid chromatography (HPLC), with reverse phase (RP-HPLC) being the preferred technique (MANT & HODGES, 1991), using a linear gradient of 1% trifluoroacetic acid (TFA) in acetonitrile and water. The purified peptides can be analyzed for their chemical homogeneity by amino acid analysis (MACHADO et *al.,* 2004) .

**Table 1. List of synthetic peptides and sequences thereof.**

| **Compou nd** | **Molecul ar Mass** | **Sequence** | **SEQ ID NO:** | **Number of amino acids** |
|---|---|---|---|---|
| **D3** | 1271.3 | DYWHDGHDLN | 1 | 10 |
| **L1** | 1132.3 | IWTKDGKAIT | 2 | 10 |
| **L2** | 1198.3 | NYFKNGKDVN | 3 | 10 |
| **L3** | 1201.5 | VWRKNGKKVS | 4 | 10 |
| **L4** | 1202.4 | TWNKKGKKVN | 5 | 10 |
| **L5** | 1292.5 | SWLKNGREFR | 6 | 10 |
| **L6** | 1258.4 | QPEISWTRNK | 7 | 10 |
| **L8** | 1257.4 | RWLKDGQAFH | 8 | 10 |
| **P4** | 1310.5 | YAIGYSCKDYK | 9 | 11 |
| **E8** | 824.9 | YSIVAGCL | 10 | 8 |
| **Cambly** | 5856.4 | EEQTHFHFESPKLISFKVQDYWILN DIMKKNLTGISLKSEEEDADSGEID | 11 | 50 |
| **E8-Cambly** | 6875.5 | YSIVAGSLGGGGEEQTHFHFESPKL ISFKVQDYWILNDIMKKNLTGISLK SEEEDADSGEID | 12 | 62 |
| **P4-Cambly** | 7360.9 | YAIGYSSKDYKGGGGEEQTHFHFES PKLISFKVQDYWILNDIMKKNLTGI SLKSEEEDADSGEID | 13 | 65 |

### Cell cultivation

The present invention includes the culture of monocytic cells of human leukemia (THP-1), osteoclast, primary human blood-derived mononuclear cells (PBMNC), and human chondrocytes (NHAC-kn).

The cells were grown in a specific culture medium according to their characteristics and needs at 37°C in an incubator containing 5% CO₂.

### Differentiation of THP-1 in Macrophages

Macrophages were obtained from the differentiation of monocytes THP-1. Briefly, 1 × 10⁷ cells were treated in the RPMI medium with 25 nM PMA for 48 hours followed by 24 hours of rest in medium without PMA. Subsequently, the cells were released and plated for the respective treatments.

### Neuronal differentiation obtained from SHSY5Y cells

The differentiation process consists of 6 stages distributed over 7 days. On the first day of the differentiation protocol (day 0), cells were seeded in collagen-coated plates. On days 1, 3, and 4 the culture medium was removed, and the differentiation medium containing 2% fetal bovine serum (FBS) and retinoic acid (RA) 10 µM) was added. On day 5, the medium was removed and the culture was incubated with culture medium and brain-derived neurotrophic factor (BDNF) (50 ng/mL). On day 7 of the differentiation process, differentiated neurons are fully ready for use (Agholme *et al.,* 2010, with some modifications) .

### Osteoclasts cultivation

### Experimental Model

Figure 1 shows an experimental model of osteoclast differentiation for the screening of synthetic peptides A. Positive controls and C. Negative differentiation controls. B. Influence of synthetic peptides (200 nM) on osteoclast differentiation.

### Chondrocyte cultivation

The primary human chondrocytes used in this assay were purchased from Lonza: Normal Human Articular Primary chondrocytes (NHAC-Kn, #CC-2550, Lot 8F3336). Chondrocyte cell culture was performed in bottles (Sarstedt T175 and T75). The cells were cultivated in DMEM culture medium (Sigma) supplemented with 10% fetal bovine serum (FBS) (Sigma), incubated in an oven at 37°C and 5% CO₂, and cultures were used in the sixth passage. The culture medium was changed every two days until the culture reached 80% confluence.

### Cell viability

The evaluation of metabolically active cells was performed using the colorimetric assay of [3-(4,5-dimethylthiazol-2-yl)-2,5-diphenyl tetrazolium] bromide (MTT, Sigma-Aldrich, St. Louis, USA), as previously reported (Twentyman *et al*., 1987). After each incubation period, the supernatant was removed and a MTT solution (0.5 mg/mL in phosphate-buffered saline - PBS) was added, and the cells incubated at 37°C and 5% CO₂ for 3 hours (Yang *et al.,* 2012) . After this period, the solution was removed and crystals of dark blue formazan were solubilized with 1 mL of dimethyl sulfoxide (DMSO, Merck, Darmstad) for 5 minutes. Quantification of formazan was done using an automatic ELISA microplate reader (SLT, Austria) at 570 nm.

### Anti-nociceptive / anti-inflammatory model

7 × 10³ neurons/well were incubated with 10 µM of morphine (inhibitory response control) or peptides (200 nM and 500 nM) 3 hours before or 3 hours after incubation with IL-1β (10 ng/mL) or glycated collagen (GC) (100 µg/mL) (both positive controls for the release of substance P). After 24 hours of incubation, the cell viability assay and the detection of substance P and β-endorphin in the supernatant were performed.

### Multiplex with magnetic spheres (MILLIPLEX^{®} MAP Magnetic Bead Panel)

The kit is based on Luminex^{®} xMAP^{®} technology that allows the simultaneous analysis of various cytokines. The technique is based on marked magnetic fluorescent spheres, known as MagPlex^{®}-C microspheres. These microspheres are covered with capture antibodies specific for IL-6 myokines, and specifically capture the analyte in the supernatant. Each microsphere was individually identified and the results were analyzed using Luminex^{®} Xponent^{®} 4.2 acquisition software.

### TRAP+ cell count

PBMNC were isolated and, immediately after, plating inductive culture medium (composed of Alpha-MEM + 10% FBS + MCSF [25 ng/mL] + RANKL [50 ng/mL] + TGF-beta 1 [5 ng/mL] + Dexamethasone [1 µM]) was added for 15 days. On day four, the *L. obliqua* venom at a concentration of 0.5/mL or toxins at a concentration of 200 nM, each individually, was added to the osteoclast precursor culture and these culture conditions were maintained until the end of the differentiation. Then, the cells were fixed and stained with tartrate-resistant acid phosphatase (TRAP).

### Results

### Osteoclast

### TRAP+ cells count

Figure 1 shows an experimental design of the osteoclast differentiation model and the screening of anti-inflammatory peptides.

Figure 2 shows the count of TRAP-positive cells observed after the peptide treatments. Using statistical analysis, the number of TRAP+ cells after the incubation of osteoclast precursors with synthetic peptides was evaluated. Figure 2 shows the summary data from three independent experiments. L2 and P4 showed no statistically significant difference in the number of TRAP+ cells, while a significant decrease in TRAP+ cells was observed after treatment with E8-Cambly when compared to the positive control (CTRL).

### THP-1

### THP-1 Multiplex

In the multiplex assay, D3 and L4 peptides showed an anti-inflammatory effect on all inflammatory cytokines tested, significantly reducing the levels of IL-1β, IL-6, and TNF-α when compared to the group treated only with LPS. If, on the one hand, D3 also reduced levels of IL-10, the only anti-inflammatory cytokine tested, L4 promoted an increase in this cytokine (Figure 3).

Figure 3 shows the anti-inflammatory effects of the synthetic peptide in the production of inflammatory (IL-1β, IL-6, and TNF-α) and anti-inflammatory (IL-10) cytokines. THP-1 cells differentiated into macrophages were induced with LPS (10 ng/mL) and, one hour later, treated with synthetic peptides, 200 nM dexamethasone (inflammation inhibitor, 10 µM). After 24 hours, the culture medium was collected and IL-1β (A), IL-6 (B), TNF-α (C), and IL-10 were measured by Multiplex. * p < 0.05 vs. LPS; ** p < 0.01 vs. LPS; *** p < 0.005 vs. LPS.

The peptides with the most promising anti-inflammatory effects, D3 and L4, were then tested on a dose-response curve. The 200 nM dose of L4 was enough to achieve the greatest inhibition in the release of inflammatory cytokines concerning IL-6 and IL-8, while 1 µM was the most effective dose concerning TNF-α and IL-23 (Figure 4). Regarding the release of cytokines tested with D3, the best dose, in all cases, was 1 µM (Figure 4). Both L4 and D3 reduced the production of inflammatory cytokines in response to LPS in a dose-dependent way.

Figure 4 shows the dose-response effect of L4 and D3 peptide on the production of inflammatory cytokines. THP-1 cells differentiated into macrophages were induced with LPS (10 ng/mL) and, one hour after, treated with L4 (0.05 µM, 0.2 µM, 1 µM, and 2 µM). After 24 hours, the culture medium was collected and TNF-α, IL-6, IL-23, and IL-8 were measured by Multiplex. (A) L4 peptide. (B) D3 peptide.

### SH-SY5Y

### Cell viability and production of inflammatory/nociceptive mediators

Regarding cell viability, there was no cytotoxic effect of peptides on neurons. Considering IL-1β as a positive control, when peptides were added 3 hours before IL-1β, 200 nM of D3 peptide significantly inhibited the production of substance P induced by IL-1β (Figure 5). No significant differences were detected in the production of β-endorphin. In the assays in which the peptides were added 3 hours after IL-1β, 200 nM of D3 was able to inhibit the production of substance P and induced the production of β-endorphin (Figure 5).

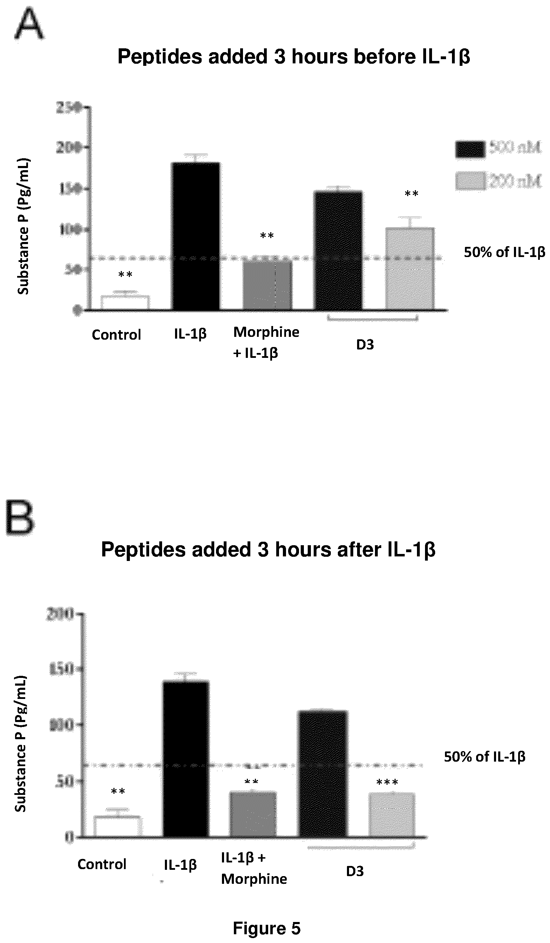

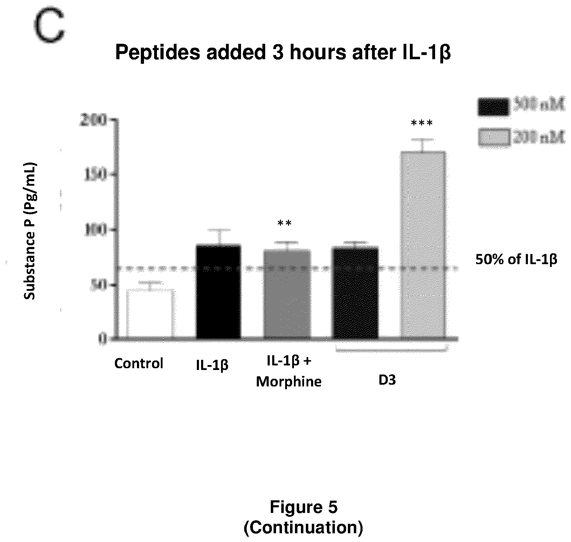

Figure 5 shows (A) production of Substance P in neurons incubated with peptides 3 hours before incubation with IL-1β; (B) Production of Substance P; (C) Production of β-endorphin in neurons incubated with peptides 3 hours after incubation with IL-1β. ANOVA and Dunnet ** p < 0.01, *** p < 0.001 compared to IL-1β.

Using the glycated collagen (GC) matrix as a positive stimulus, when the peptides were incubated 3 hours before the GC, the Cambly peptide was able to significantly decrease the production of Substance P (Figure 6), however, it did not affect the production of β-endorphin. On the other hand, when the peptides were incubated 3 hours after GC, 200 nM of P4, D3, Cambly, E8, and P4-Cambly, significantly inhibited the production of Substance P and increased the production of β-endorphin (Figure 7A).

Figure 6 shows the production of substance P in neurons incubated with peptides, 3 hours before incubation with GC. ANOVA and Dunnet * p < 0.05; ** p < 0.01 compared to GC.

Figure 7 shows: (A) Production of Substance P; (B) Production of β-endorphin in neurons incubated with peptides, 3 hours after incubation with GC. ANOVA and Dunnet * p < 0.05 ** p < 0.01, *** p < 0.001 compared to GC.

### Chondrocytes

### Multiplex

The identification and quantitative analysis of the cytokines present in the peptide treatment supernatant were performed using the Luminex xMAP Milliplex MAP Human Cytokine / Chemokine Magnetic Bead Panel Catalog #HCYTOMAG-60K technology, according to the manufacturer's instructions.

Six analytes that demonstrated changes in the presence of cytokine IL-1β (1 ng/mL), some deserve to be highlighted since they also have their presence altered from the treatment with the peptides: G-CSF, GM-CSF, IL-6, IL-8, MCP-1, and TNF-α (Figure 8).

This study identified several cytoprotective peptides with an anti-inflammatory effect. Among the peptides with anti-inflammatory activity are D3, P4, E8, L1, L2, L3, L4, L5, L6, and L8.

Hemopoietic CSF, granulocyte-macrophage CSF (GM-CSF), and granulocyte CSF (G-CSF) are cytokines that mediate clonal proliferation and differentiation of progenitor cells into mature macrophages and/or granulocytes. In rheumatoid disease, chondrocytes are known as a potential source of CSF. The present invention proposes that the production of chondrocytes in the CSF in response to IL-1, and the simultaneous destruction of cartilage by IL-1, could provide a mechanism for the chronic nature of rheumatoid arthritis.

Figure 8 shows the effect of the peptides on the release of six analytes. Values were obtained from each analyte in pg/mL from treatment with the peptides in the presence of IL-1β (1 ng/mL). The data are presented as the mean ± MSE (*p < 0.05 when compared to the control with IL-1β, test t, n = 3).

The P4-Cambly peptide is a hybrid molecule containing the 10 amino acid sequence of the P4 peptide and a 50 amino acid sequence known as Cambly or CTER. The peptide sequence called "CTER" corresponds to a peptide derived from the Amblyomin-X molecule (Batista *et al.,* 2010) with characteristics of CPP (cell-penetrating peptides), known in the literature as molecules that transport a wide variety of biologically active conjugates("charges") to cells (Guidotti *et al.,* 2017). Thus, CPPs are an innovative way for a drug to reach intracellular targets, reducing concentrations that could be toxic. The patent for this sequence (CTER) and its use as a CPP is being developed.

### Conclusions

**THP-1:** D3 and L4 peptides reduced the production of inflammatory cytokines in response to LPS and both acted in a dose-dependent way.

The P4 peptide has no effect on the viability of macrophages derived from THP-1. However, it has the ability to inhibit part of the LPS-stimulated activation suggesting an anti-inflammatory action.

**SH-SY5Y Neurons:** D3 and Cambly peptides inhibited the production of substance P when added before the positive stimulus.

D3, P4, E8, Cambly, P4-Cambly peptides inhibited the production of substance P and increased the production of β-endorphin when added after the positive stimulus (glycated collagen matrix).

**Osteoclast:** Cambly and E8-Cambly peptides reduced the number of TRAP+ cells.

**Chondrocytes:** The data of the present invention are aligned with other models, suggesting that P4, E8, and D3, as well as the L1, L2, L3, L4, L5, L6, and L8 derived peptides, are potential candidates for the search for new targets for inflammation.

The peptides tested in the THP-1, Synoviocytes, and Chondrocytes models showed a significant reduction in the production of important cytokines for the inflammation process of various inflammatory diseases, such as arthritis. Besides, the peptides were also able to decrease pain markers in the neuron model.

### PHARMACEUTICAL COMPOSITION

In another aspect of the present invention, the pharmaceutical compositions provided herein comprise said anti-inflammatory peptides and at least one pharmaceutically acceptable carrier.

The term "pharmaceutically acceptable carrier" includes, but is not limited to, any carrier that does not interfere with the effectiveness of the biological activity of the active ingredients and that is not toxic to the patient to whom it is administered. Examples of suitable pharmaceutical carriers are well known in the art and include phosphate-buffered saline solutions, water, emulsions, such as oil/water emulsions, various types of wetting agents, sterile solutions, etc. Such a carrier can be formulated by conventional methods and can be administered to the individual in the dose and therapeutic routines most appropriate to each case. Preferably, the compositions are sterile, but can also be prepared under aseptic conditions. These compositions can also contain adjuvants, preservatives, emulsifying agents, and dispersing agents. Pharmaceutical formulations can be for a human, and/or veterinary / animal use. The formulations may be prepared in the form of particulate systems such as microparticles, nanoparticles, microspheres, nanospheres, liposomes, in carrier complexes as in cyclodextrins (alpha, beta, and gamma); still in the form of controlled release. The formulations can be prepared by associating the peptides in the most appropriate proportions and doses/concentrations. The formulations can be in the form of creams, ointments, gels, adhesives, sprays, including nanotechnology-based formulations, or even through the association with other active principles of the same biological effect, or even through the association with antimicrobials, antifungals, etc.

The administration of the compositions of the present invention can be carried out in different pathways, for example, intravenous, subcutaneous, intramuscular, topical or intradermal, particularly, the main ones are cutaneous, subcutaneous, topical, intradermal, rectal, intraocular, nasal, oral and auricular. The administration pathway, of course, depends on the type of treatment and the type of compound contained in the pharmaceutical composition. The dosage routines will be determined by the doctor and other clinical factors. As already well known in medical techniques, dosages for any patient depend on many factors, including the patient's weight, body area, age, sex, as well as pharmaceutical composition carrying the particular compound to be administered, time and administration pathway, type of therapy, general health status and other factors to be simultaneously considered.

### USE OF THE PHARMACEUTICAL COMPOSITIONS OF THE PRESENT INVENTION

The present invention also relates to the use of a synthetic peptide for the preparation of a pharmaceutical composition for cell remodeling, such as in reducing inflammation and in degenerative diseases of an inflammatory nature, in which the degenerative diseases of an inflammatory nature include rheumatoid arthritis and osteoarthritis.

The present invention also relates to the use of the synthetic peptide and/or pharmaceutical composition comprising said peptide as an agent that induces cell proliferation, preferably in tissue repair.

Thus, although only a few embodiments of the present invention have been shown, it will be understood that various omissions, substitutions, and changes in the pharmaceutical composition comprising the anti-inflammatory peptide and their uses can be made by a person skilled in the art, without departing from the spirit and scope of the present invention.

It is expressly provided that all combinations of elements that perform the same function in substantially the same way to achieve the same results are within the scope of the present invention. Substitutions of elements from one embodiment described to another are also fully intended and contemplated.

It is also necessary to understand that the drawings are not necessarily to scale, but that they have only a conceptual nature. Therefore, the intention is to be limited, as indicated by the scope of the attached claims.

### REFERENCES

AKKIRAJU, H., NOHE, A. Role of Chondrocytes in Cartilage Formation, Progression of Osteoarthritis, and Cartilage Regeneration. Journal of developmental biology. 2015; 3: 177-192.

ALVAREZ-FLORES, M. P., FRITZEN, M., REIS, C. V., CHUDZINSKI-TAVASSI, A. M. Losac, a factor X activator from Lonomia obliqua bristle extract: its role in the pathophysiological mechanisms and cell survival. Biochem Biophys Res Commun. 2006 May 19;343(4): 1216-23.

FRITZEN, M.; SCHATTNER, M.; RIBEIRO, A. L. Q.; BATISTA, I. F. C.; VENTURA, J.; PREZOTO, B. C. & CHUDZINSKI-TAVASSI, A. M. A prothrombin activator (Lopap) modulating inflammation, coagulation and cell survival mechanisms. Biochemical and biophysical research communications, v. 333, n. 2, p. 517-523, 2005.

GOLDRING, M. B.; OTERO, M. Inflammation in osteoarthritis. Current opinion in rheumatology, v. 23, n. 5, p. 471, 2011.

HE, X.; HUANG, L.; QIU, S.; YIN, X.; SHEN, Y.; WU, Y.; JIANG, Y.; FANG, J. β-Endorphin attenuates collagen-induced arthritis partially by inhibiting peripheral pro-inflammatory mediators. Exp Ther Med. 15(4): 4014-4018, 2018.

KAPOOR, M.; MARTEL-PELLETIER, J.; LAJEUNESSE, D.; PELLETIER, J. P.; FAHMI, H. Role of proinflammatory cytokines in the pathophysiology of osteoarthritis. Nature Reviews Rheumatology. Jan. 7 (1): 33, 2011.

LEE, K.; SEO, I.; CHOI, M. H.; JEONG, D. Roles of Mitogen-Activated Protein Kinases in Osteoclast Biology. Int J Mol Sci. 19(10): 3004, 2018.

MACHADO, A.; LIRIA, C. W.; PROTI, P. B.; REMUZGO, C.; MIRANDA, M. Chemical and enzymatic synthesis of peptides: basic principles and applications. New Chemistry. 24(5): 781-789, 2004.

MANT, C. T.; HODGES, R.S. High-performance Liquid Chromatography of Peptides and Proteins: Separation, Analysis, and Conformation, CRC Press Inc.: Boca Raton, 1991.

MOSMANN, T. Rapid colorimetric assay for cellular growth and survival: application to proliferation and cytotoxicity assays. Journal of Immunological Methods; v. 65, n 1-2, p. 55-63, 1983.

NI, Z., KUANG, L., CHEN, H., XIE, Y., ZHANG, B., OUYANG, J., WU, J., ZHOU, S., CHEN, L., SU, N., TAN, Q., LUO, X., CHEN, B., CHEN, S., YIN, L., HUANG, H., DU, X., CHEN, L. The exosome-like vesicles from osteoarthritic chondrocyte enhanced mature IL-1β production of macrophages and aggravated synovitis in osteoarthritis. Cell Death Dis. 10(7): 522, 2019.

PERROT, S. Osteoarthritis pain. Best practice & research Clinical rheumatology, v. 29, n. 1, p. 90-97, 2015.

REIS, C. V.; PORTARO, F. C. V.; ANDRADE, S. A.; FRITZEN, M.; FERNANDES, B.; SAMPAIO, C. A. M.; CAMARGO, A. C. M. & CHUDZINSKI-TAVASSI, A. M. A prothrombin activator serine protease from the Lonomia obliqua caterpillar venom (Lopap): biochemical characterization. Thromb. Res. 102: 427-36, 2001a.

REIS, C. V., FARSKY, S. H. P., FERNANDES, B. L., SANTORO, M. L., OLIVA, M. L. V., MARIANO, M., CHUDZINSKI-TAVASSI, A. M. In vivo characterization of Lopap, a prothrombin activator serine protease from the Lonomia obliqua caterpillar venom. Thromb. Res. 102:437-43, 2001b.

SPROUSE-BLUM, A. S.; SMITH, G.; SUGAI, D.; PARSA, F. D. Understanding endorphins and their importance in pain management. Hawaii Med J. 69(3): 70-1, 2010.

ZANNIN, M.; LOURENÇO, D. M.; MOTTA, G.; DALLA COSTA, L. R.; GRANDO, M.; GAMBORJE, G.; NOGUTI, M. A. & CHUDZINSKI-TAVASSI, A. M. Blood coagulation and fibrinolytic factors in 105 patients with hemorrhagic syndrome caused by accidental contact with Lonomia obliqua caterpillar in Santa Catarina, southern Brazil. Thromb. Haemost. 89:355-364, 2003.

ZHOU, P. H.; LIU, S. Q; PENG, H. The effect of hyaluronic acid on IL-1β-induced chondrocyte apoptosis in a rat model of osteoarthritis. Journal of Orthopedic Research. Jun 3; 26 (12): 1643-1648, 2008.

## Claims

1. Synthetic peptides **characterized in that** it comprises the amino acid sequences selected from the group comprising D3 (SEQ ID NO: 1), L1 (SEQ ID NO: 2), L2 (SEQ ID NO: 3), L3 (SEQ ID NO: 4), L4 (SEQ ID NO: 5), L5 (SEQ ID NO: 6), L6 (SEQ ID NO: 7), L8 (SEQ ID NO: 8), P4 (SEQ ID NO: 9), E8 (SEQ ID NO: 10), Cambly (SEQ ID NO: 11), E8-Cambly (SEQ ID NO: 12) and P4-Cambly (SEQ ID NO:: 13).

2. Pharmaceutical composition **characterized in that** it comprises one of the synthetic peptides as defined by claim 1 and a pharmaceutically acceptable carrier, conveyer, excipient.

3. Pharmaceutical composition, according to claim 2, **characterized in that** the composition is for intravenous, subcutaneous, intramuscular, topical, oral, or intradermal administration.

4. Use of the synthetic peptide as defined by claim 1 or the composition as defined by claim 2, **characterized in that** it is for the preparation of a drug for cell remodeling, such as reducing inflammation and degenerative diseases of an inflammatory nature, in which the degenerative diseases of an inflammatory nature include rheumatoid arthritis and osteoarthritis.

5. Use, according to claim 4, **characterized in that** the D3 and L4 peptides reduce the production of inflammatory cytokines, such as TNF-α, IL-6, IL-23, and IL-8, in response to LPS in the model of macrophages derived from THP-1, and both act in a dose-dependent way.

6. Use, according to claim 5, **characterized in that** the D3, L3, and L4 peptides significantly reduce the levels of IL-1β, IL-6, and TNF-α in the model of macrophages derived from THP-1.

7. Use, according to claim 4, **characterized in that** the P4 peptide has the ability to inhibit part of the LPS-stimulated activation acting in an anti-inflammatory action in the model of macrophages derived from THP-1.

8. Use, according to claim 4, **characterized in that** the L4, P4, and L5 peptides have the ability to significantly increase IL-10 levels in the model of macrophages derived from THP-1 stimulated by LPS, acting in anti-inflammatory action.

9. Use, according to claim 4, **characterized in that** the peptides are able to decrease pain markers in the neuron model.

10. Use, according to claim 4, **characterized in that** the D3 and Cambly peptides inhibit the production of substance P when added before the positive stimulus in the neuron model.

11. Use, according to claim 4, **characterized in that** the D3, P4, E8, Cambly, and P4-Cambly peptides inhibited the production of substance P and increase the production of β-endorphin when added after the positive stimulus (glycated collagen matrix) in the neuron model.

12. Use, according to claim 4, **characterized in that** the Cambly, E8, and E8-Cambly peptides significantly decrease the number of positive cells for TRAP (tartrate-resistant acid phosphatase), indicating a reduction in the number of osteoclasts and an anti-inflammatory effect.

13. Use, according to claim 4, **characterized in that** the P4, E8, and D3 peptides reduce the inflammatory cytokines production, such as TNF-α, IL-6, and IL-8, after 24 hours of stimulation with IL-1β in the chondrocyte model.

14. Use, according to claim 4, **characterized in that the** P4, E8, and D3 peptides reduce the production of stimulators of proliferation and differentiation of hematopoietic precursors: G-CSF, and GM-CSF, after 24 hours of stimulation with IL-1β in the chondrocyte model.

15. Use, according to claim 4, **characterized in that** the L1, L2, L4, L5, L6, and L8 peptides have the ability to reduce IL-6 production after 24 hours of stimulation with IL-1β in the chondrocyte model.
